# EUROPEAN PATENT APPLICATION

(11) **EP 3 797 779 A1**
(43) Date of publication of application: **31.03.2021**
(21) Application number: 19382836.5
(22) Date of filing: 30.09.2019
(51) Int. Cl.: A61K 31/59, A61K 39/00, A61P 13/12

(54) **COMBINED THERAPY COMPRISING AN INHIBITOR OF INTERLEUKIN-17 ACTIVITY AND A VITAMIN D RECEPTOR AGONIST**

(71) Applicant: Servei de Salut de Les Illes Balears - Ibsalut, 07120 Palma de Mallorca (Islas Baleares) (ES); Fundación para la Investigación Biomédica del Hospital Universitario Ramón y Cajal, 28034 Madrid (ES)
(72) Inventor: Uriol Rivera, Miguel Giovanni, 07120 PALMA DE MALLORCA (ES); Gómez Marques, Gonzalo, 07120 PALMA DE MALLORCA (ES); Carrillo Garcia, Paula, 07120 PALMA DE MALLORCA (ES); Obrador Mulet, Aina Remei, 07120 PALMA DE MALLORCA (ES); Delgado Sanchez, Olga, 07120 PALMA DE MALLORCA (ES); Gomez Lobon, Ana, 07120 PALMA DE MALLORCA (ES); Julià Benique, María Ros, 07120 PALMA DE MALLORCA (ES); Daza Cajigal, Vanessa Cerisse, 07120 PALMA DE MALLORCA (ES); Garcia Alvarez, Angel, 07120 PALMA DE MALLORCA (ES); Luque Ramirez, Manuel, 28290 LAS MATAS - LAS ROZAS DE MADRID (ES); Nattero Chavez, Lia, 28290 LAS MATAS - LAS ROZAS DE MADRID (ES)
(74) Representative: ZBM Patents - Zea, Barlocci & Markvardsen

(57) **Abstract**

The invention relates to a combined therapy including an inhibitor of interleukin-17 (IL-17) activity for use in combination with a vitamin D receptor agonist in the prevention and/or treatment of diseases or conditions known as autoimmune and autoinflammatory concomitant diseases, such as autoimmune kidney diseases, autoimmune joint diseases, skin autoimmune diseases, autoimmune neurological diseases, autoimmune thyroid diseases, and gout disease. The invention provides also the combination for use in the treatment and/or prevention of cancer, in which deregulation of immune system takes place. The invention also relates to the therapeutic combination and to its use in the treatment or prevention of proteinuria.

## Description

### Technical Field

Present invention relates to the field of preventing and treating autoimmune and autoinflammatory diseases, in particular those being refractory to common treatments.

### Background Art

Autoimmune diseases are a group of diseases of high complexity, in several of which autoinflammatory processes may occur, that lead to chronic inflammation of tissues. Generically, in autoimmune diseases (ADs) the innate immune system activates, against self-antigens, the adaptive immune system which, in turn, is also responsible for the inflammatory process.

Autoinflammation is a process that takes place in a group of diseases known as autoinflammatory diseases (AIDs), in which the innate immune system directly causes tissue inflammation. AIDs and ADs are characterized by a chronic activation of immune system, which eventually leads to tissue inflammation in genetically predisposed individuals.

There seems to be a narrow relationship between autoimmune and autoinflammatory diseases, and recently it has been discovered that in traditionally considered autoimmune diseases (i.e. psoriasis, systemic lupus, inflammatory bowel disease, rheumatoid arthritis, etc.), autoinflammatory processes plays also a critical role.

One cytokine playing an important role in chronic inflammation (for example in Chron's disease) that occurs during the pathogenesis of autoimmune diseases and allergies is interleukin-17 (IL-17). However, contradictory effects are linked to this cytokine. Based on the biologic functions and regulation, IL-17 has regulatory roles in host defence and chronic inflammation, which result in tissue damage and autoimmunity. So, the IL-17 links innate and adaptive immunity, but it has also been seen that IL-17 has both beneficial and pathological effects on the immune system.

IL-17 is a cytokine whose gene was isolated from a rat-mouse T cell hybridoma in 1993. Since it displayed a high degree of homology with the HVS13 Herpes virus gene, it was thought to be a subtype of the cytotoxic T-lymphocyte-associated protein (CTLA) family of proteins and called CTLA-8. In 1995, it was recognized as a new cytokine and named IL-17, and, today, six homologous molecules are known (IL-17A through IL-17F). To date, most studies are focused on IL-17A, IL-17E, and IL-17F. IL-17A and IL-17F, which are highly homologous and share receptors. Thus, they have extremely similar functions. IL-17E is also known as IL-25, and because it presents low homology with other molecules of the family and contributes to the induction of allergies, it is thought to have functions different from those of IL-17A. Several types of immune cells produce IL-17A, and Th17 cells, the newly established subset of helper T cells, have received particular attention.

Blocking IL-17A activity by means of an specific monoclonal antibody recognizing it, secukinumab (Cosentyx® of Novartis Europharm Ltd), was ineffective and higher rates of adverse events were noted compared with placebo in a clinical trial including patients with Crohn's disease (see for example Wang et al., "Rapid onset of inflammatory bowel disease after receiving secukinumab infusion" ACG case Rep J, 2018 5: p. e56.). Secukinumab is a fully human IgG1/^{k} monoclonal antibody that selectively binds to and neutralises the proinflammatory cytokine IL-17A. Secukinumab targets IL-17A and inhibits its interaction with IL-17 receptor expressed on various cell types. As a result, the release of proinflammatory cytokines is reduced.

On the other hand, secukinumab did was effective in a case of refractory lupus nephritis in a patient suffering from psoriasis and systemic lupus erythematosus, as explained by Satoh et al., "A case of refractory lupus nephritis complicated by psoriasis vulgaris that was controlled with secukinumab", Case Report Lupus (2018) 0, 1-5. The authors administered secukinumab after realising of the presence of Th17 cells in peripheral blood and the presence of IL-17 positive lymphocytes infiltrated in renal tissue, to which presence the worsening of some parameters in the patient were correlated. In this particular case the authors observed that after secukinumab administration, the number of activated Th17 cells was reduced and renal function improved. Interestingly, however, was that when secukinumab was administered proteinuria increased, although previously with methylprednisolone it has been reduced. This proteinuria increase (ratio urine protein/creatinine (Up/Cre) demonstrates that blocking IL-17 is dangerous, at least momently, for the kidney.

A recent report published by Masahiko Ochi et al, showed a negative effect of the use of the IL-17 inhibitor in one patient with psoriasis and IgA nephropathy in proteinuria, and steroids pulses and tonsillectomy were needed to control the nephrotic syndrome, which confirms that the inhibition of the IL-17 alone, was associated with a negative effect on proteinuria when this treatment is administered alone. (See Ochi et al., "A case of secondary IgA nephropathy accompanied by psoriasis treated with secukinumab", CEN Case Rep-2019 Apr 3. doi: 10.1007/s13730-019-00393-5).

This primary dangerous effect if an IL-17 inhibitor is in concordance with the fact that in several kidney diseases, such as in experimental glomerulonephritis or in diabetic nephropathy, the role of IL-17 was revealed as good and, therefore the blocking or inhibition of IL-17 would not be recommended at all in kidney diseases (see Hamour et al, Local IL-17 Production Exerts a Protective Role in Murine Experimental Glomerulonephritis, PlosOne 2015. 10(8): p.e0136238; and Galvan et al., Paradoxical Role of IL-17 in Progression of Diabetic Nephropathy, J Am. Soc. Nephrol 2016. 27(3): p. 657-8.).

Another indicia that IL-17 may have a positive role rather than promoting kidney injury is derived from Rosendhal et al., "Adaptative immunity and IL-17A are not involved in the progression of chronic kidney disease after 5/5 nephrectomy in mice", Br J Pharmacol. 2018 Sep 30. doi: 10.1111/bph.14509. Rosendhal et al. showed in an IL-17 deficient mice (IL-17A-/-) to which chronic kidney disease was simulated after 5/6 nephrectomy, that albuminuria increased throughout the experiment. They concluded that in contrast to many other experimental models of renal injury, genetic deficiency of IL-17 did neither attenuate induction nor progression of chronic kidney disease was simulated after 5/6 nephrectomy.

Among the examples of complex autoimmune and autoinflammatory concomitant diseases, Crohn's disease and immunoglobulins A nephropathy are highly impeding diseases, supposing high costs to health authorities and also to society since they are common in working-age population. Other complex autoimmune and autoinflammatory concomitant diseases include autoimmune thyroid diseases (i.e. Grave's disease and Hashimoto's thyroiditis). Another highly impeding disease with indicia of being an autoimmune and autoinflammatory concomitant disease is the neurological disease multiple sclerosis.

Immunoglobulins A nephropathy (IgAN) is the most prevalent primary glomerulonephritis. Prognostic is good but about 30 % of the patients will likely develop an advanced kidney disease that will need of chronic substitutive kidney therapy (dialysis or kidney transplantation). Evolution of the IgAN is followed by determining hematuria, proteinuria and arterial blood pressure. Main control of IgAN is performed by means of corticosteroid therapy, renin angiotensin system inhibitors, as well as immunosuppressive therapy (i.e mycophenolate) and biologic compounds (i.e. *Rituximab,* a chimeric monoclonal *antibody* targeted against the pan-B-cell marker CD2); unfortunately, without controversial results.

Despite the treatments mentioned previously, some patients are refractory to therapy, and some kidney transplanted patients previously suffering from IgAN finally relapse. IgAN is considered a multi-cause or multi-hit disease comprising an autoimmune component but also other factors. The autoimmune component mainly derives from abnormalities in mucosal immunity and is well known the presence of elevated amounts of a galactose deficient IgA (Gd-IgA), the presence of autoantibodies recognizing Gd-IgA, and the presence of circulating immune complexes containing the Gd-IgA (complexes form of autoantibodies and of Gd-IgA). These complexes are deposited in glomeruli, finally damaging this structure due to the recruitment of pro-inflammatory cells and cytokines (See. Wu et al., "The emerging role of pathogenesis of IgA nephropathy", Journal of Clinical Medicine-2018, vol. no. 7, 225).

The glomerular deposit of Gd-IgA and the immune complexes induces the permanent and increasing mesangial proliferation cells, which are the responsible for the disruption of the glomerular filtration barrier (GFB) with two different implications:
- The onset of hematuria. In normal health status the endothelium fenestrations (50-100 nm) are too small in comparison with red blood cells (RBC) diameter (6.2-8.2 µm) which maintain the RBCs away from the glomerular basal membrane (GBM). Hematuria results in heme group release in the glomeruli with high toxic effects, denature cellular proteins and altering cellular integrity and also increasing inflammation through nuclear factor Kappa beta (NF-kb) pathway.

As a result, it is hypothesized that a decrease of the immune complexes depositions may restore GFB, stopping hematuria and its toxics and inflammatory effects.

A very important issue in the IgAN is the permanent inflammation state. Traditionally, a few numbers of syndromes are known as autoinflammatory diseases (AIDs). As previously indicated, these diseases consist in acute and recurrent inflammation episodes. IgAN has been reported associated with some genes altered leading disorders in the innate immune response. Interestingly, in some patients with one AIDs known as Familial Mediterranean Fever, the IgAN has been described, and also in other AIDs named hyper IgE syndrome the plasma levels of the Immunoglobulin A are elevated which suggest that AIDs are associated with the IgAN.

Recently, the gout disease is considered an AID, this fact is critical because elevated plasma uric acid (hyperuricemia) is the main biochemical disorders. In IgAN patients, hyperuricemia is a predictor for mortality and for the risk of the renal failure. The mortality risk is independent of the renal function, condition that suggests that hyperuricemia "per se" indicates systemic disorders. It is important to remark, physiologic levels of uric acid are the predominant anti-oxidant molecule in plasma and is necessary and sufficient for induction of type 2 immune responses. The uric acid acts as a danger signal promoting the generation of inflammatory monocyte-derived dendritic cells. The uric acid results from the metabolism of the purines derived from damage, dyeing and dead cells and are typically increased in chronic renal disease patients and it has been associated with mesangial and proximal tubular cells damage. Thus, a therapy that decreases plasma uric acid represents a potential benefit not only for preventing the direct mesangial and tubular cells and also because lowering plasma uric acid may be associated with a normalized type 2 immune response. The type 2 immune disorders are recently described as an initial pathogenic step which alters intestinal epithelial barrier.

Therefore, although there are treatments that face several of the symptoms of these considered multi-cause (or multi-hit) diseases, there is still a need of more effective while safe therapies.

On the other side, the main sign of renal damage is proteinuria (excess of serum proteins in urine), that occurs when serum proteins are not reabsorbed from urine due to an insufficiency of absorption or an impaired filtration. Proteinuria is a feature of nephrotic syndromes (intrinsic renal failure), toxic lesions of kidney, glomerular diseases, stress, diabetes mellitus, and some infections (HIV, post-streptococcal infection, hepatitis), among other conditions. Since it finally supposes a stress for kidney, it is highly desired to find methods directed to control proteinuria.

Finally, it is also known that certain neoplasms can trick the immune system or any immunotherapy trying to destroy malignant cells. Although many are the therapies for cancer, additional approaches are always welcomed.

### Summary of Invention

The invention provides, as a first aspect, a combination comprising:
a) an inhibitor of interleukin-17; and b) a vitamin D receptor agonist.

Inventors have surprisingly found that the combination in a sequential manner of a selective vitamin D receptor activator, in particular paricalcitol and subsequently an inhibitor of IL-17 activity, in particular the monoclonal antibody secukinumab, are associated with a significantly reduction in proteinuria in relation to initial basal proteinuria in IgAN patients, indicating that the introduction in a sequential manner of the paricalcitol and subsequently the secukinumab may restore the filtration proteins process at the glomeruli; while if the secukinumab is used alone or in different IgAN glomerular diseases, the proteinuria may dramatically worsen.

Moreover, the sequential treatment was also capable to restore the glomerular filtration barrier, fact explained by the sequential therapy where hematuria completely disappeared in IgAN patients, indicating an important benefit related to the immunocomplexes deposition due to autoimmune component control.

IgAN is considered as a multi-cause disease catalogued as an autoimmune disease; however, the clinical and pathophysiologic effects observed in the IgAN and previously disclosed in detail in the background, induced the inventors to assumption that the IgAN is indeed a dual disease with autoimmune and autoinflammatory concomitant disease and conceived a particular treatment protocol that surprisingly allowed controlling autoimmune and autoinflammatory components with extremely beneficial results.

To avoid potential damage induced by the IL-17 blocking, as was reported in several case reports, inventors surprisingly found that by inducing in the patient an anti-inflammatory milieu increasing the expression of the IL-10 and inhibiting the Th1 cells prior to blocking IL-17, any adverse effect induced by IL-17 blocking was actually avoided. Moreover, it is known that the administration of inhibitors of the IL-17 are associated with a decrease in the expression of the Treg lymphocytes. The Treg are T cells that modulate the immune system, maintain tolerance to self-antigens, and prevent autoimmune disease, so, a lowering number of Treg is also an undesirable effect of the IL-17 blocking.

As will be illustrated below, treatment with a selective Vitamin D receptor agonist prior to IL-17 blocking or inhibition, represents an effective and safety therapeutic schedule for the prevention and/or treatment of several diseases catalogued as autoimmune and autoinflammatory concomitant disease.

Inhibitors of IL-17 activity have caused in several cases serious adverse effects, as illustrated in the case of Crohn's disease, another multi-cause disease considered an autoimmune disease with a concomitant autoinflammatory component, as previously disclosed. Therefore, the positive effect observed now by inventors with the sequential treatment was really unexpected.

Without being bound to any theory, inventors consider that when administered in combination with a vitamin D receptor agonist, in particular with a selective vitamin D receptor agonist; a context of anti-inflammatory environment is achieved allowing reducing any adverse effect that could be caused directly or indirectly by the inhibitor of IL-17 activity.

Examples below relate thus to the combination therapy including secukinumab and paricalcitol, which make theoretically plausible that any adverse effect that could be caused by any inhibitor of IL-17 activity in a disease, can be reduced or avoided if it is administered in combination with any vitamin D receptor agonist.

Another surprising effect observed by inventors was that of a change of immunophenotype of the Th1 lymphocytes, or which is the same, a change of the set of proteins that are expressed by this cell type. Inhibition of IL-17 activity as well as of Th17 and Treg blockade provide harmful effects (glomerulonephritis and colitis) if this blockade is accompanied by the presence and complete activation of Th1. This change of phenotype would make that recruitment of Th1 due to blockade of IL-17 be less negative in terms of adverse side-effects or even to supress any of them because Th1 cells do not act as proinflammatory actors. Th1 is a type of T helper cells that mainly produce interferon-gamma, essential for macrophage activation and clearance of pathogens, but they are also involved in cell-mediated autoimmune diseases (see. Turner et al., "The Th17 immune response in renal inflammation", Kidney International -2010, vol. no. 77, pp.: 1070-1075).

Even another surprising effect observed by the inventors, and contrary to what was expectable, was an increase in urine of endogenous lysosomal capacity of kidney cells, determined by a two-fold increase of lysosomal enzyme N-acetyl-beta-D-glucosaminidase but with a not kidney deleterious effect, as observed with the accompanying proteinuria percentage reduction. Thus, more than a marker of renal damage, as usually accepted, when found in conjunction with a reduction of proteinuria in relation to basal values before any treatment, this enzyme also indicates that there is a local increase in lysosomal activity with the consequence of a high autophagy capacity in a controlled mode not involving kidney damage. Therefore, it is herewith disclosed the combination of proteinuria and of N-acetyl-beta-D-glucosaminidase urine levels, in an isolated urine sample of a subject, as marker of kidney healing or of non-damage of kidney due to administration of an inhibitor of IL-17 activity.

The compounds of the invention may be formulated in different types of compositions/kits of parts. Thus, the invention encompasses also, as a second aspect, a package or kit of parts comprising:
i) a first pharmaceutical or veterinary composition which comprises an amount of an inhibitor of IL-17 as defined in any of claims 1-7, together with one or more pharmaceutically or veterinary acceptable excipients or carriers; and
ii) a second pharmaceutical or veterinary composition which comprises an amount of vitamin D receptor agonist, together with one or more pharmaceutically or veterinary acceptable excipients or carriers;
wherein the first and second compositions are separate compositions, and wherein the amount of the compound of inhibitor of IL-17 of i) and the amount of vitamin D receptor agonist of ii) in combination are therapeutically effective.

The combination of the invention is for use as a medicament.

Further, as mentioned above, the combination of the invention may be used in autoimmune and concomitant autoinflammatory diseases or conditions.

Thus, a fourth aspect of the invention relates to the combination or a package or kit of parts as defined above, for use in the treatment and/or prevention of an autoimmune and concomitant autoinflammatory disease or condition (also termed in this description as synonymous of the term autoimmune disease or condition with autoinflammatory concomitant component)

This later aspect may also be formulated as a method of treatment and/or prevention of an autoimmune and concomitant autoinflammatory disease or condition, which comprises administering to a mammal subject in need thereof, including a human subject, either
a) a therapeutically effective amount of the combination comprising a) an inhibitor of interleukin-17; and b) a vitamin D receptor agonist; or alternatively
b) the package or kit of parts as defined above.

It also forms part of the invention the use of a combination comprising: (a) an inhibitor of interleukin-17; and (b) a vitamin D receptor agonist; for the preparation of a medicament for the treatment and/or prevention of an autoimmune and concomitant autoinflammatory disease or condition.

Based on observations in thyroid cancer, the combination or a package or kit of parts as defined above is also proposed to be used as immunotherapy in the prevention and/or treatment of malign neoplasia.

Thus, another aspect of the invention is a combination or a package or kit of parts as defined above, for use in the treatment and/or prevention of a cancer, optionally with metastatic behaviour, in which deregulation of immune system takes place.

The sequential administration of a Vitamin D receptor agonist, in particular a selective Vitamin D receptor activator, such as paricalcitol, with in vitro evidence of an antitumoral effect, and an inhibitor of interleukin-17 activity inhibits tumor progression by restoring the immune response against tumoral cells and by blocking pathogenic mechanisms favoring tumor immune tolerance.

This aspect may also be formulated as a method of treatment and/or prevention of a cancer, optionally with metastatic behaviour, in which deregulation of immune system takes place, which comprises administering to a mammal subject in need thereof, including a human subject, either
a) a therapeutically effective amount of the combination comprising a) an inhibitor of interleukin-17; and b) a vitamin D receptor agonist; or alternatively
b) the package or kit of parts as defined above.
It also forms part of the invention the use of a combination comprising: (a) an inhibitor of interleukin-17; and (b) a vitamin D receptor agonist; for the preparation of a medicament for the treatment and/or prevention of a cancer, optionally cancer with metastatic behaviour, in which deregulation of immune system takes place.

Finally, another aspect of the invention is a combination or a package or kit of parts as defined above, for use in the treatment and/or prevention of proteinuria.

With the proposed combination therapy, the cocktail of cytokines involved in a pathological condition involving tumor immune tolerance, or autoimmunity and autoinflammation concomitant conditions, is modulated in such a way that anti-inflammatory environment is first promoted with the vitamin D receptor agonist, and then inhibition of pro-inflammatory items are blocked, reduced or effectively minimized in a safe way with the inhibitor of IL-17 activity. Thus, the proposed combination of actives works as a multi-cytokine normalization levels therapy (abbreviated by inventors as MNL). It is in particular useful in those pathological conditions cursing with exacerbation of Th17 and Th1 cells, being this exacerbation the main cause of the disease or one of them. The proposed combination therapy provokes an increasing of lysosomal activity in kidney cells, being useful then in all those diseases where this technical effect can suppose a way to reduce inflammation and a way to eliminate accumulation of dangerous immunocomplexes or dangerous protein residual compounds accumulated in the cytosol of cells.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

For "inhibitor of interleukin-17 (IL-17) activity" or "IL-17 inhibitor" or "inhibitor of IL-17", used indistinctly as synonymous in this description, is to be understood any agent, either a compound, mixture of compounds or cells, capable of blocking IL-17 activity by directly contacting the cytokine and avoiding it can take contact with the receptor sited in several cells (e.g. Th17 cells), or indirectly by blocking the receptor of IL-17. As will be illustrated below particular inhibitors are compounds capable of blocking, totally or at some extent or particular inhibitory concentrations at certain inhibition percentage (i.e. IC50), IL-17 interaction with its cell receptors. Particular inhibitors are antibodies (monoclonal or polyclonal) and fragments of these antibodies that specifically recognize IL-17. This activity of IL-17 can be determined by any known method, in which IL-17 is involved, such as for example the presence or absence in a tissue of inflammatory cells, such as Th17, neutrophils, monocytes, Th1 cells (e.g. measured by flux cytometry), and/or the presence or absence of further proinflammatory cytokines (e.g. CXCL1, CCL2, CCL20) downstream the signal cascade measured using immunoassays (ELISA).

The expression "formulated for sequentially administration" or "sequentially" as appears in this description means that both drugs are not administered simultaneously, but within a spaced time. This spaced time can be of hours among the same day, or of days between administration of one of inhibitor of IL-17 activity and the vitamin D receptor agonist.

The expression "therapeutically effective amount" as used herein, refers to the amount of a compound that, when administered, is enough to prevent development of, or alleviate to some extent, one or more of the symptoms of the disease which is addressed. The expression "therapeutically effective" as used herein throughout this description, refers to the amount of a compound or combination of compounds that, when administered, is enough to prevent development of, or alleviate to some extent, one or more of the symptoms of the disease which is addressed. In this particular description, it is the amount of a compound, combination of compounds, or composition that produces a desired therapeutic effect in a subject, such as treating autoimmune and concomitant autoinflammatory diseases or conditions, or cancer, optionally with metastatic behaviour, in which deregulation of immune system takes place.

The precise therapeutically effective amount is an amount of the composition that will yield the most effective results in terms of therapeutic efficacy in a given subject. The specific dose of the compound of the invention to obtain a therapeutic benefit may vary depending on the particular circumstances of the individual patient including, among others, the size, weight, age and sex of the patient, the nature and stage of the disease, the aggressiveness of the disease, and the route of administration. The specific dose of the compound of the invention to obtain a therapeutic benefit when administered in said combinations, compositions or kit of parts, may vary in relation with the specific dose of the compound used as single active agent.

Those particular embodiments of the combinations above, are also applicable as particular embodiments of the packages or kits of parts of the invention.

The expression "pharmaceutically or veterinary acceptable excipients or carriers" refers to pharmaceutically or veterinary acceptable materials, compositions or vehicles. Each component must be pharmaceutically acceptable in the sense of being compatible with the other ingredients of the pharmaceutical or veterinary composition. It must also be suitable for use in contact with the tissue or organ of humans and animals without excessive toxicity, irritation, allergic response, immunogenicity or other problems or complications commensurate with a reasonable benefit/risk ratio.

### Combinations of the invention

As above indicated, the first aspect of the invention is a combination comprising:
a) an inhibitor of interleukin-17; and b) a vitamin D receptor agonist.

Several inhibitors of IL-17 can be used. In a particular embodiment of the combination, the inhibitor of IL-17 is an antibody or a fragment of said antibody, both of which specifically recognize one or more epitopes of a mammal IL-17, including human IL-17, and being capable of blocking IL-17 activity.

In another particular embodiment, the inhibitor of IL-17 activity is an inhibitor of any of the members of the IL-17 family: IL-17A, IL-17B, IL-17C, IL-17D, IL-17E and IL-17F. IL-17E is also known as IL-25. More in particular, it is an inhibitor of IL-17A. In mammals, the sequences of these cytokines are highly conserved and have a high percentage of identity. In a more particular embodiment, optionally in combination with any embodiment above or below, the inhibitor is an inhibitor of mammal IL-17 A, more in particular an inhibitor of human IL-17A. Human IL-17A corresponds to protein sequence identified with UniProtKB database accession number Q16552-1, version 1 of November 1, 1996. It has a length of 155 amino acids and it is further processed into a mature form.

In a more particular embodiment, the inhibitor of IL-17 is secukinumab or a fragment thereof, said fragment specifically recognize one or more epitopes of human IL-17 A and being capable of blocking IL-17 A activity. Among fragments there are included Fab, F(ab')2, Fc, and combinations of them.

The inhibitor of IL-17 (i.e. secukinumab) may be administered at a dose comprised from 75 mg/week to 300 mg/week, which doses are therapeutically effective.

Skilled person in the art is able to calculate doses of particular inhibitors of IL-17 with their own particular knowledge and considering guidance for the effect disclosed above.

According to this description a "vitamin D receptor agonist" or "Vitamin D receptor activator" (used as synonymous) includes any compound capable of interacting with vitamin D receptor (VDR) and to provoke an affect as active metabolite of Vitamin D does. Vitamin D₃ is made in the skin and modified in the liver and kidney to form the active metabolite, 1,25-dihydroxyvitamin D₃ (calcitriol). Calcitriol binds to a nuclear receptor, the vitamin D receptor, and activates VDR to recruit cofactors to form a transcriptional complex that binds to vitamin D response elements in the promoter region of target genes. Several VDR agonists (VDRAs) have been developed for the treatment of osteoporosis, psoriasis and hyperparathyroidism secondary to chronic kidney disease (CKD). VDRAs are well known for their capacity to control calcium and bone metabolism and to regulate growth and differentiation of many cell types. In a particular embodiment of the invention, this vitamin D receptor agonist is selected from the group consisting of cacitriol (CAS number 32222-06-3), paricalcitol (CAS number 131918-61-1), calcipotriene, tacalcitol, ercalcitriol (CAS number 60133-18-8) any pharmaceutically or veterinary acceptable salt or ester thereof, and combinations thereof. Among vitamin D receptor agonists, particular ones are the known as "selective Vitamin D receptor activators", which act as agonists but have minimal undesirable effects on calcium absorption in the intestine, and calcium and phosphorus mobilisation in the bone compared with nonselective VDRAs. Thus, in a particular embodiment, optionally in combination with one or more features of the various embodiments and aspects described above or below throughout all the description, the vitamin receptor agonist is a selective VDRA.

In even a more particular embodiment, the vitamin D receptor agonist is paricalcitol or a pharmaceutically or veterinary acceptable salt or ester thereof. Paricalcitol is a selective VDRA, with a low hypercalcemia induction in relation with other nonselective VDRAs, and for this reason is a more particular embodiment. Paricalcitol allows obtention of the desired effect of receptor activation without the disadvantageous increase of calcium in blood. Paricalcitol is a therapeutic compound, classified as an antiparathyroid compound.

The vitamin D agonist (i.e. paricalcitol) may be administered at a dose comprised from 0.4 micrograms/day (mcg/day) to 2 mcg/day, which doses are therapeutically effective. Even more particular doses are comprised from 0.4 mcg/day to 1.0 mcg/day, and more in particular f from 0.70 mcg/day to 2.0 mcg/day. Other more in particular doses are from 0.75 mcg/day to 1 mcg/day. 0.75 mcg/day is a safety and low enough dose. Noteworthy, doses of 0.75 mcg/day and lower (0.4 mcg/day-0.75 mcg/day) suppose being using a known antiparathyroid compound with a new indication, namely as an anti-inflammatory compound with these lower doses.

In another particular embodiment, optionally in combination with one or more features of the various embodiments described above or below throughout all the description, the combination comprises:
a) an inhibitor of IL-17, and
b) a vitamin D receptor agonist,
wherein the amount of a) and the amount of b) in combination are therapeutically effective.

This means that both amounts of (a) and (b) can be separately amounts considered therapeutically effective, or in the alternative, subtherapeutically effective amounts but therapeutically effective given in combination due to the therapeutic schedule.

In a more particular embodiment of the combination of the first aspect, optionally in combination with one or more features of the various embodiments described above or below throughout all the description, the vitamin D receptor agonist is the selective VDRA, paricalcitol, and the inhibitor of IL-17 activity is secukinumab.

### Package or kit of parts

The present invention also relates to a package or kit of parts comprising:
i) a first pharmaceutical or veterinary composition which comprises an amount of an inhibitor of IL-17 activity as defined in any of claims 1-7, together with one or more pharmaceutically or veterinary acceptable excipients or carriers; and
ii) a second pharmaceutical or veterinary composition which comprises an amount of vitamin D receptor agonist, together with one or more pharmaceutically or veterinary acceptable excipients or carriers;
wherein the first and second compositions are separate compositions, and wherein the amount of the compound of inhibitor of IL-17 of i) and the amount of vitamin D receptor agonist of ii) in combination are therapeutically effective.

Those particular embodiments of the combinations above, are also applicable as particular embodiments of the packages or kits of parts of the invention.

Thus, present invention also relates, in a more particular embodiment, to a combination comprising an inhibitor of IL-17 activity and a vitamin D receptor agonist, wherein both are provided in a packaging comprising a support with separate compartments, each compartment comprising one of the inhibitor of IL-17 activity and a vitamin D receptor agonist, said packaging comprising schedule means for appliance of a prescribed dosage regimen.

The phrases "dose" and "dosage" are used interchangeably herein.

The election of the pharmaceutical or veterinary formulation will depend upon the nature of the active compound and its route of administration. Any route of administration may be used, for example oral, parenteral and topical administration.

For example, the pharmaceutical or veterinary composition may be formulated for oral administration and may contain one or more physiologically compatible carriers or excipients, in solid or liquid form. These preparations may contain conventional ingredients such as binding agents, fillers, lubricants, and acceptable wetting agents. In a particular embodiment of the combinations of the invention, the vitamin D receptor agonist is for oral administration.

The pharmaceutical or veterinary composition may be formulated for parenteral administration in combination with conventional injectable liquid carriers, such as water or suitable alcohols. Conventional pharmaceutical or veterinary excipients for injection, such as stabilizing agents, solubilizing agents, and buffers, may be included in such compositions. These pharmaceutical or veterinary compositions may be injected subcutaneously, intramuscularly, intraperitoneally, or intravenously. In a particular embodiment of the combinations of the invention, the inhibitor of IL-17 activity is for subcutaneous administration.

The pharmaceutical compositions may be in any form, including, among others, tablets, pellets, capsules, aqueous or oily solutions, suspensions, emulsions, or dry powdered forms suitable for reconstitution with water or other suitable liquid medium before use, for immediate or retarded release.

The appropriate excipients and/or carriers, and their amounts, can readily be determined by those skilled in the art according to the type of formulation being prepared.

### Treatment of autoimmune and concomitant autoinflammatory disease or condition, treatment of cancer and treatment of proteinuria

It also forms part of the invention a combination, or a package or kit of parts as defined above, for use in the treatment and/or prevention of an autoimmune disease or condition with autoinflammatory concomitant component (autoimmune and autoinflammatory concomitant disease).

Indeed, inventors have realized as depicted below that the proposed combination is, in particular, for use in the treatment and/or prevention of any metabolic alteration, more in particular uric acid levels in plasma, proteinuria and insulin resistance in autoimmune diseases.

In a particular embodiment of the combination or kit of parts for use as defined above, optionally in combination with one or more features of the embodiments above or below, the autoimmune disease or condition with autoinflammatory concomitant component is selected from an autoimmune kidney disease, an autoimmune joint disease, a skin autoimmune disease, an autoimmune neurological disease, an autoimmune thyroid disease, and gout disease.

As previously indicated, IgAN is considered as a multi-cause disease catalogued as an autoimmune disease; however, the clinical and pathophysiologic effects observed in the IgAN and previously disclosed in detail in the background, induced the inventors to assumption that the IgAN is indeed a dual disease with autoimmune and autoinflammatory concomitant disease. As will be detailed in examples surprising results were observed in patients to which the combination of the invention was applied.

Among the autoimmune kidney disease or condition, particular one is nephropathy IgA. In even a more particular embodiment the disease is IgA nephropathy, in particular refractory to other treatments for IgA nephropathy or as a relapse of the disease after kidney transplantation.

As depicted in examples, patients treated with the proposed combination therapy experienced a high reduction of proteinuria, in relation to basal value before therapy. Therefore, present description proposes this combination therapy for use in the treatment of the symptoms of kidney damage or of kidney disease. Although treating proteinuria may not result in the treatment of the main cause of the disease, even more when several are the causes involved in the disease, at least the treating of proteinuria will minimize the worsening of the pathological condition.

In another particular embodiment, the combination or kit of parts is for use in the prevention and/or treatment of proteinuria in a subject suffering from a kidney autoimmune and autoinflammatory disease.

In another particular embodiment, the combination or kit of parts is for use in the prevention and/or treatment of an autoimmune neurological disease, more in particular is for use in the prevention and/or treatment of multiple sclerosis.

In another particular embodiment, the combination or kit of parts is for use in the prevention and/or treatment of a gut disease, which is Chron's disease.

In another particular embodiment, the combination or kit of parts is for use in the prevention and/or treatment of an autoimmune thyroid disease, which is selected from hypothyroidism, Hashimoto's thyroiditis, hyperthyroidism, and Grave's disease.

The control of the Th1, Th17 is considered as a potential treatment in autoimmune thyroid disease. Autoimmune thyroid diseases such as Grave's disease and Hashimoto's thyroiditis are closely related from a pathogenic point of view. In both, environmental factor and genetic susceptibility interact leading to a humoral and cellular immune response against thyroid antigens. Th1 CD4+ lymphocytes predominate in thyroid tissue of patients with Hashimoto's disease, and there is solid evidence that regulatory mechanisms of immune cells are disturbed in those patients. The biochemical control of hypothyroidism in patients with Hashimoto's thyroiditis is straightforward. Nonetheless, there is a substantial percentage of biochemically well-controlled patients who complaint of persisting and life-limiting symptoms to the extreme to be willing to be operated. That persisting symptoms is thought to be related to autoimmunity rather than to hormonal dysfunction. A systemic inflammatory reaction, the presence of cross-reactivity between anti-thyroid peroxidase antibodies (anti-TPO) and other antigens, and/or the coexistence of rheumatic autoimmune diseases. This surgery procedure is based on normalizing levels of serum anti-TPO antibodies, thereby reducing inflammation and alleviating symptoms, by means of a complete removal of the antigenic tissue via total thyroidectomy. Furthermore, the proportion of Th17 cells in patients with intractable Graves's disease appears to be higher than in those patients in remission, and seems to be implicated in the inflammatory response, disease activity, and fibrosis of the thyroid-associated ophtalmopathy (TAO) too. Thus, the immunomodulation as a result of the sequential treatment with a Vitamin D receptor agonist, in particular a selective Vitamin D receptor activator and an inhibitor of interleukin-17 activity, such as is proposed in the present application will promote:
- A detention of the chronic inflammatory process in thyroid gland, and a potential partial/complete recovery at initial stages of the Hashimoto's disease.
- An improvement in persisting symptoms despite a good biochemical control in patients with Hashimoto's thyroiditis.
- A potential therapeutic option in patients with TAO.
- A potential therapeutic option in patients with Grave's disease refractory to conventional treatments.

In another particular embodiment, the combination or kit of parts is for use in the prevention and/or treatment of an autoimmune joint disease, which is rheumatoid arthritis. Other more systemic autoimmune diseases include systemic lupus.

In another particular embodiment, the combination or kit of parts is for use in the prevention and/or treatment of an autoimmune skin disease, which is psoriasis.

The combination, or the package or kit of parts as defined above is also for use in the treatment and/or prevention of a cancer, optionally with metastatic behaviour, in which deregulation of immune system takes place.

In other words, is for use in the treatment of neoplasms, particularly with metastasis, as immune therapy to avoid tolerance to tumor of subject's immune system.

In a particular embodiment, the cancer thyroid cancer (also named thyroid carcinoma).

The presence of tumor-associated lymphocytes and lymphocytic thyroiditis is frequently found in the histopathologic reports of patients with follicular epithelium-derived thyroid cancer. The former likely contribute to tumor tolerance favoring cancer cell proliferation. Th17 cells are an important mediator in inflammation-associated cancer: i) patients with thyroid tumors appears to have a higher proportion of Th17 and lower proportion of Tc17 cells in peripheral blood; ii) several polymorphisms in IL-17 receptor (IL17R) are associated with the development of papillary thyroid cancer; and iii) high IL-17 expression in tumoral tissues is associated with a worse prognosis in terms of recurrence and mortality.
But Th17 immunologic-mediated pathways may promote or inhibit tumor progression. Therefore, the sequential administration of a selective Vitamin D receptor activation, such as paricalcitol, with in vitro evidence of an antitumoral effect, and an inhibitor of interleukin-17 activity is inhibiting tumor progression by restoring the immune response against tumoral cells and by blocking pathogenic mechanisms favoring tumor immune tolerance.

The combination, or a package or kit of parts as defined above, is also for use in the treatment and/or prevention of proteinuria, in particular in any disease or condition cursing with this symptom.

In another particular embodiment, optionally in combination with one or more features of the embodiments above or below, the combination of IL-17 inhibitor and vitamin D receptor agonist, or the package or kit of parts as defined above, both the inhibitor of IL-17 and vitamin D receptor agonist are formulated for first administration of the vitamin D receptor agonist for a period of time comprised from 2 to 4 weeks before administration of the inhibitor of IL-17.

This means that the inhibitor of IL-17 activity is for use in combination with a vitamin D receptor agonist in the prevention and/or treatment of the above listed diseases or conditions, and wherein said inhibitor of IL-17 and vitamin D receptor agonist are formulated for sequentially administration of a therapeutically effective amount of any of them. In particular, they are formulated for first administration of the vitamin D receptor agonist and second the administration of the inhibitor of IL-17 activity after a period in which vitamin D receptor agonist has been previously administered.

In even a more particular embodiment, optionally in combination with one or more features of the aspects and embodiments above or below, the combination or a package or kit of parts are for use in the diseases and conditions previously disclosed, and both inhibitor of IL-17 and vitamin D receptor agonist are formulated for sequentially administration according to the following schedule:
- a daily dose of vitamin D receptor agonist; and
- a weekly dose of inhibitor of IL-17.

In yet another also more particular embodiment of the combination, or the package or kit of parts as defined above, the inhibitor of IL-17 is for use subcutaneously and the vitamin D receptor agonist is for use orally.

As previously disclosed, particular doses of any of the compounds in the combination or kit of parts, may be adjusted according to skilled man knowledge. In a particular embodiment of the combination, or the package or kit of parts as defined above, for use in the diseases and conditions previously disclosed, the daily dose of vitamin D receptor agonist is comprised from 0.4 mcg/day to 2 mcg/day, and the weekly dose of inhibitor of IL-17 activity is comprised from 75 mg/week to 300 mg/week. These are doses including those approved, in particular, for paricalcitol and secukinumab for human use.

In another particular embodiment, optionally in combination with embodiments above or below, the dose of vitamin D receptor agonist is from 0.70 mcg/day to 2.0 mcg/day. In a more particular embodiment is from 0.70 mcg/day to 1.0 mcg/day. More in particular, vitamin D receptor agonist is the selective vitamin D receptor activator paricalcitol with a dose from 0.70 mcg/day to 2.0 mcg/day, even more in particular from 0.70 mcg/day to 1.0 mc/day, and even more in particular a dose from 0.75 mcg/day to 1.0 mcg/day.

In another particular embodiment, optionally in combination with embodiments above or below, the dose of inhibitor of IL-17 activity is of 300 mg/week. More in particular, inhibitor of IL-17 activity is secukinumab with a dose from 75 mg/week to 300 mg/week, even more in particular a dose from 100 mg/week to 300 mg/week.

In a more particular embodiment, the daily dose of vitamin D receptor agonist is for use for a period of time of at least 6 weeks. Generally, the use of the vitamin D receptor agonist is conceived as a chronic treatment, which means that the subject is receiving the compound along life, optionally with resting periods in which no treatment is administered. The time of these resting periods, if any, are scheduled by prescriber (i.e. doctor).

In yet another particular embodiment, the weekly dose of inhibitor of IL-17 activity is for use for a period of time from at least 5 weeks. More in particular is of 300 mg/week. In a more particular embodiment, after the weekly dose of inhibitor of IL-17 activity for a period of at least 5 weeks, the administration schedule further includes a monthly dose of inhibitor of IL-17 activity for a period of at least 6 months.

As indicated for the use of the vitamin D receptor agonist, also the use of inhibitor of IL-17 activity is conceived as a chronic treatment, which means that the subject is receiving the compound along life, optionally with resting periods in which no treatment is administered. The time of these resting periods, if any, are scheduled by prescriber (i.e. doctor).

In even a more particular embodiment, both inhibitor of IL-17 and vitamin D receptor agonist are formulated for sequentially administration according to the following schedule:
- a daily dose of 1 mcg/day of vitamin D receptor agonist, in particular paricalcitol, at least 4 weeks before a first weekly dose of the inhibitor of IL-17 activity; and
- a weekly dose of 300 mg/week of inhibitor of IL-17 activity, in particular secukinumab, for a period of at least five weeks, and optionally a monthly dose of 300 mg for at least six months.

As depicted in examples, patients treated with the proposed combination therapy experienced a high reduction of proteinuria, in relation to basal value before therapy. Therefore, present description proposes this combination therapy for use in the treatment of the symptoms of kidney damage or of kidney disease. Although treating proteinuria may not result in the treatment of the main cause of the disease, even more when several are the causes involved in the disease, at least the treating of proteinuria will minimize the worsening of the pathological condition.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

### BIGART STUDY (Balear Immunoglobulin A Research Treatment)

In order to describe the invention BigART study detailed as follow was carried out:
A. Inclusion criteria:
   1.- *Biopsy-proven* IgA nephropathy.
   2.- Age older than 18 years.
   3.- Prior treatment with steroids.
   4.- Prior treatment with an inhibitor of a angiotensin converted enzyme.
   5.- Prior treatment with any immunosuppressive drug (mycophenolate, anticalcineurin in particular tacrolimus).
   6.- Proteinuria > 0,5 g/24hours.
   7.- Signed informed consent.
   8.- Chronic kidney disease stage 1-3 according EPI-CKD algorithm.
B. Exclusion criteria:
   1.- Active inflammatory intestinal disease.
   2.- Serum calcium > 10,5 mg/dl.
   3.- Serum phosphate > 5 ng/ml.
   4.-Active infection
C. Determinations
   - Changes during the follow -up in proteinuria g/l and proteinuria in 24h.
   - Changes during the follow-up in diastolic and systolic blood pressure.
   - Changes during the follow-up in plasma uric acid.
   - Changes during the follow up in plasma creatinine levels.
   - Changes during the follow-up in urine N-Acetyl galactosaminidase.
D. Efficacy criterion
   Decrease in >= 30% of the proteinuria respect to baseline.
E. Time of follow-up
   The treatment consists of two phases:
   a) Induction Phase: from month 0 to month 1.
   b) Maintenance Phase: from month 1 to month 6.
F. Schedule of determinations:
   Months 0, month 1, 3 and 6.
G. Treatment Schedule:
   a) Induction phase
      Paricalcitol 0.4 to 1 mcg/day at least one month [given in assay exactly 1 mcg/day] Combined treatment:
      Paricalcitol 0.4 to 1mcg/day [given in assay exactly 1 mcg/day] plus Secukinumab 300 mg/ weekly. Doses administered at week: 0,1,2,3 y 4.
   b) Maintenance phase:
      Paricalcitol 0.4 mcg to1 mcg/day [given in assay exactly 1 mcg/day].
      Secukinumab 300mg/monthly during 6 months.

In any of the induction phase (a) and maintenance phase (b), some patients could equally have received a dose of paricalcitol from 0.4 to 1mcg/day. Indeed, in any of the induction phase (a) and maintenance phase (b), other patients (data not shown) received an average of 0.71 mcg/day of paricalcitol (5 days of treatment in a week at a dose of 1 mcg/day of paricalcitol). This dose of paricalcitol gave also good results.

### RESULTS:

**Basal data. Table A**

| | SEX | AGE | PRIOR TREATMENT | COMORBIDITY |
|---|---|---|---|---|
| 1 | Male | 31 | Prednisone, *Mycophenolate,* enalapril | |
| 2 | Male | 31 | Prednisone,*Mycophenolate*,enalapril | |
| 3 | Male | 58 | Irbesartan, prednisona, *Mycophenolate.* | Hashimoto's Thyroiditis |
| 4 | Female | 59 | Prednisone, tacrolimus, *Mycophenolate*, losartan. | |
| 5 | Male | 61 | Valsartan, metotrexate, prednisone | Ankylosing *spondylitis and diabetic nephropathy with non nephrotic proteinuria.* |

| | | | | |
|---|---|---|---|---|
| Patient 1 and patient 2 is the same in two time points. | | | | |

### I. RENAL FUNCTION EVOLUTION DURING THE FOLLOW-UP

Next Table I shows the evolution of the renal function defined as creatinine plasma levels. The variation from baseline (month 0: M0) en every patient and in every time point was neither clinically nor analitycally significant (< 0.3mg/dl). It means that the sequential therapy (called also multi-cytokine normalized levels_MNL in this description) was safety and moreover the treatment stabilizes de renal function. This aspect will be take into a count because the lysosomal activity determined by urine N-acetyl-Beta-D-glucosaminadase showed an initial increase, which was related in the prior art as a negative effect associated with a renal damage. As a whole the mean values of the plasma creatinine (Table VIII), confirms that the renal function did not decline over the time which is one of the main objetive of the sequential tretament.

**Table I. Renal function evolution**

| Patient | Creatinine (mg/ml) | Months |
|---|---|---|
| 2 | 2.1 | -3 |
| | 2.1 | 0 |
| | 2.2 | 1 |
| | 2.1 | 3 |
| | 2.3 | 6 |
| 3 | 1.4 | -3 |
| | 1.7 | 0 |
| | 1.7 | 1 |
| | 1.7 | 3 |
| | 1.8 | 6 |
| 4 | 1.4 | -3 |
| | 1.7 | 0 |
| | 1.7 | 1 |
| | 1.7 | 3 |
| | 1.8 | 6 |
| 5 | 1.3 | -3 |
| | 1.3 | 0 |
| | 1.5 | 1 |
| | 1.5 | 3 |
| | 1.2 | 6 |

**Table VIII. Renal function evolution as a whole during follow-up, excluding patient 5**

| Creatinine (mg/ml) | Months |
|---|---|
| 2.0 | 0 |
| 2.0 | 1 |
| 2.0 | 3 |
| 2.1 | 6 |

### II. PROTEINURIA EVOLUTION DURING THE FOLLOW-UP

Table II shows the evolution of the proteinuria determined by its quantification in 24-hours colleted urine. In data of patient 1 initial increase in the proteinuria level was observed, however it decreased progresively at month 3 and 6. The paitent 1, 2 and 3 decreased the 24 hours proteinuria in 62%, 44% and 58% respectively, which is clinically significant.

**Table II. Proteinuria evolution during the follow-up**

| Patient | Urine protein (g/24h) | Months |
|---|---|---|
| 1 | 2.1 | 0 |
| | 2.8 | 1 |
| | 0.8 | 3 |
| | 0.8 | 6 |
| 2 | 3.2 | 0 |
| | 1.1 | 1 |
| | 1.3 | 3 |
| | 1.8 | 6 |
| 3 | 4.3 | 0 |
| | 2.3 | 1 |
| | 2.3 | 3 |
| | 1.8 | 6 |
| | | |
| 4 | 1.8 | 0 |
| | 2.0 | 1 |
| | 1.9 | 3 |
| | 3.5 | 6 |
| | | |
| 5 | 2.8 | 0 |
| | 4.6 | 1 |
| | 5.0 | 3 |
| | 6.1 | 6 |

Two important considerations are necessary to remark:
- The evolution of the proteinuria in the patient number 4, not only showed a decrease in proteinuria level but increased the value at the end of the study. Conversely to patients 1, 2 and 3 the renal lysosomal activity did not increase during the induction phase, moreover, total plasma proteins finally decreased at the end of the study which indicates a bad outcome. Because the different evolution in the patient 4 respect to patients 1,2 and 3, a new renal biopsy was performed. The new renal biopsy indicated that no mesangial IgA deposits were present. The initial renal biopsy in patient 4 showed a weak IgA deposit. Revaluating the case, no elevated prior plasma IgA was observed and with the results of the new biopsy, the inventors could conclude that patient 4 was not really a IgAN patient, which may explain why the treatment failed.
- As was described previously, patient 5 was included as a control nephropathy patient. That patient was treated with secukinumab for his reumatologic disease (Ankylosing *spondylitis)* as is indicated by data sheet. However, the proteinuria levels increased progressively and significantly. The moderate proteinuria level increased to a nephrotic level (> 3,5 g/24h) which is a life-threatening condition. It was the reason why secukinumab needs to be stopped. Interestingly, this patient only received secukinumab and no paricalcitol was administered. This fact represents a crucial aspect that reinforces the invention. In other words, if no sequential treatment is administered, the only administration of secukinumab in this kind of patients may be associated with a detrimental effect as was seen in patient 5. It cannot be excluded, the fact that the diabetes may play a role in the deleterious effect on proteinuria after blocking IL-17, as was described in diabetic nephropathy model. After suspending treatment with secukinumab, proteinuria was stabilized in this patient 5. After that, paricalcitol was administered and proteinuria level was lowered. These additional data suggest the isolated administration of secukinumab in this patient was, effectively, deleterious.

The proteinuria changes during the follow-up indicates a rapid decline in their values, when it was evaluated as a whole. At the end of the induction phase, de % decrease was 28%, while the antiproteinuric effect was more pronounced in month 3 of the follow up, when lowering proteinuria reached nearly than 50%. Considering that patients were considered refractory to standar treatment, this fact represented an important tool for the proteinuria control in IgAN patients. At the end of the study the percentage decrease was 31%,that is considered as a positive result.

The previous considerations are two strong reasons for considering that the combination of the invention, package or kit of parts and their use, in particular as a sequential treatment resolves an important clinical and technical problem, turning effective and safe the IL-17 blocking.

### III. Plasma acid uric evolution

Another surprising effect discovered by the inventors is related to the uric acid (UA) evolution (See data in Table III).

**Table III. Plasma UA levels**

| Patient | Urine protein (g/24h) | Months |
|---|---|---|
| 1 | 9.3 | 0 |
| | - | 1 |
| | 7.4 | 3 |
| | 7.2 | 6 |
| 2 | 6.2 | 0 |
| | - | 1 |
| | 5.7 | 3 |
| | 7.2 | 6 |
| 3 | 7.2 | 0 |
| | - | 1 |
| | 6.4 | 3 |
| | 6.7 | 6 |
| | | |
| 4 | 6.9 | 0 |
| | - | 1 |
| | 7.1 | 3 |
| | 6.5 | 6 |
| | | |

**Table XIII. Changes in several T cells and changes in autoimmune-autoinflammatory profile**

| Patient 1 T cell type | | Months |
|---|---|---|
| T helper cells (as % of CD44 positive cells) | 40% (proteinuria 2.1 g/24h) | 0 |
| | 36% | 1 |
| | 47% | 3 |
| | - | 6 |
| T reg cells (%of cells with CD4+CD25+CD27-) | 5% | 0 |
| | 4% | 1 |
| | 5% | 3 |
| | - | 6 |
| Th17 cells (% of cells with CXCR3-CCR6+ (%CD4) | 24% | 0 |
| | 19% | 1 |
| | 12· | 3 |
| | - | 6 |
| | | |
| Autoimmune-autoinflammatory profile (Th17/Treg) | 4.8 | 0 |
| | 4.75 | 1 |
| | 2.40 | 3 |
| | - | 6 |
| | | |

Because the renal function did not change over the time, the decrease in the UA values indicates a decrease in the purine metabolism, it means a decrease in damage, dying and dead cells in the body. This was surprising, but nonetheless it could be confirmed that peripheric red blood cells and the platelets number did not decrease during the study, thus, the decrease may represent a decrease in white blood cells, in particular related to theTH17 cells evolution as is show in Table XIII. Moreover, hematuria disappeared, which is another important reason for the UA lowering effect.
The decrease of the UA was more important at the end of the induction phase, showing during the study levels considered in the normal range (3,5 to 7,2 mg/dl).

More importantly, the decrease in UA may reflect the control in the autoimmune component, because UA is necessary for the type 2 immune response, which is the direct immune disorder for treating in the sequential MNL therapy.

### IV. Plasma triglycerides levels evolution.

The triglycerides levels (See Table IV) show a desirable evolution; in particular in patient 3 and 4, who initially presented higher triglycerides levels. The explanation for its positive effect is consistent with the evolution of the proteinuria. Importantly, the benefit in blood pressure, UA and the triglycerides, keeping the same weight indicates a positive effect on the metabolic syndrome.

**Table IV. Plasma triglycerides levels during follow-up**

| Patient | Plasma triglycerides (mg/dl) | Months |
|---|---|---|
| 1 | 81 | 0 |
| | - | 1 |
| | 104 | 3 |
| | 104 | 6 |
| 2 | 116 | 0 |
| | - | 1 |
| | 116 | 3 |
| | 105 | 6 |
| 3 | 226 | 0 |
| | - | 1 |
| | 90 | 3 |
| | 95 | 6 |
| | | |
| 4 | 218 | 0 |
| | - | 1 |
| | 179 | 3 |
| | 95 | 6 |
| | | |

### V. Plasma total protein levels evolution.

According with proper control of the proteinuria, the plasma total proteins increased as shown in table V in patients 1-3. In patient 4 its values decreased progresively, indicating no response of the sequential MNL therapy in this no IgAN patient.

**Table V. Total plasma proteins**

| Patient | Total plasma protein (g/dl) | Months |
|---|---|---|
| 1 | 59.2 | 0 |
| | - | 1 |
| | 60.9 | 3 |
| | 60.9 | 6 |
| 2 | 62.2 | 0 |
| | - | 1 |
| | 70.2 | 3 |
| | 65.9 | 6 |
| 3 | 60.8 | 0 |
| | - | 1 |
| | 61.9 | 3 |
| | 63.6 | 6 |
| | | |
| 4 | 61.8 | 0 |
| | - | 1 |
| | 63.3 | 3 |
| | 60.3 | 6 |
| | | |

### VI. Renal lysosomal activity evolution

One of the most important component of the sequential MLN therapy is the controlled influence on the lysosomal activity in safe manner. During the induction phase, when combination of the selective vitamin D receptor activator and the highest inhibitor of the IL-17 antibody doses, the lysosomal activity increased in patients 1, 2 and 3; however its activity decreases in patient 4 (see data in Table VI). As a whole, the mean of the lysosomal activity determined by the urine determination of the N-Acetyl-b-D-glucoaminidase activity increased as shown in Table X. This condition informs conversely with the current state of the art, that a renal damage is not associated as the investor inform in the present application.

**Table VI. Lysosomal activity. Urine N-Acetyl-B-D-glucosaminidase U/g creatinine (U-NAG U/g creatinine) during induction.**

| Patient | U-NAG U/g creatinine | Months |
|---|---|---|
| 1 | 4 | 0 |
| | 7 | 1 |
| | - | 3 |
| | - | 6 |
| 2 | 7 | 0 |
| | 11 | 1 |
| | - | 3 |
| | - | 6 |
| 3 | 12 | 0 |
| | 14 | 1 |
| | - | 3 |
| | - | 6 |
| | | |
| 4 | 17 | 0 |
| | 9 | 1 |
| | - | 3 |
| | - | 6 |
| | | |

**Table X. Renal lysosomal activity evolution as a whole during follow-up, but excluding patient 5**

| U-NAG U/g creatinine | Months |
|---|---|
| 10.2 | 0 |
| 10.3 | 1 |
| 9.2 | 3 |
| 8.2 | 6 |

Moreover this is the first model of treatment that considers autophagy as a posible treatment in this glomerulonephritis. The inventors consider, these novel therapy may offer to the body a unique opportunity to stimulate the own enzyme mechanisms with the objective to control this deposit disease. The dg-lgA acts as a target and the sequential treatment show the capacity to induce physiologic mechanism to control the disease. Respect to the current art, another opposite but beneficial consequence of the sequential MNL therapy is that the increase in the lysosomal activity was associated with a decrease in proteinuria levels.

After the induction phase, the lysosomal activity decreased progresively in every patients and as a whole. Those changes occurred after the induction phase and was associated with a progressive decrease in the proteinuria levels. The progressive decrease on renal lysosomal activity was associated with a progressive decrease in the proteinuria levels, during that period of time the doses of the secukinumab was lower that in the induction phase; however the dosage of the paricalcitol and the secukinumab was the same which clearly indicates that the beneficial effect on proteinuria was maintained during all the maintenance phase.

In patient 4, the lysosomal activity decreased from the basal high levels, but the proteinuric response in this patient was unsatisfactory, and completely opposite in comparison with patients 1, 2 and 3. These findings inform that the sequential MNL therapy is highly selective for IgAN patients treatment. In these patients and in patients 5 (diabetic nephropathy) the use of secukinumab was associated with a detrimental effect on proteinuria, as was seen in our patient and also reported in prior art.

The lysosomal activity increased in our patients could be due to the "wipe" effect on IgA mesangial deposits. Critical data was obtained in relation with patient 4, who was included initially with a renal biopsy with IgA deposits, unexpectedly the new renal biopsy performed for no response in proteinuria informed that no IgA deposits were observed. These findings confirm the effect of the sequential MNL therapy with IgA deposition and the selective benefits in IgAN.

The progressive reduction in the proteinuria levels, even with the stable doses of the paricalcitol and secukinumab, suggests that an early benefit obtained with the treatment has been occurred. The hematuria completely disappeared at month 3, indicating that the restoring of the glomerular filtration barrier (functional benefits) occured in a short period of time while the proteinuria control was a slower process (histologic process).

### VII. Restoring glomerular filtration barrier evolution

As describe previously, hematuria is a hallmark sing of the glomerulonephritis process. In tested patients, this kind of glomerular disorder was completely restored in all patients, confirming that the immunocomplexes deposition (autoimmune component of the disease) in the mesangial area of the glomerulus improved after treatment. Moreover, the effect on hematuria was permanent indicating a clear repairing process of the glomerular filtration barrier.
This effect was observed in patients 1,2,3 and 4 and the mean change over time is showed in Table VII.

**Table VII.**

| Patient | U-NAG U/g creatinine | Months |
|---|---|---|
| 1 | 3.9 | 0 |
| | 7.2 | 1 |
| | 6.9 | 3 |
| | 6.9 | 6 |
| 2 | 6.9 | 0 |
| | 11 | 1 |
| | 14.02 | 3 |
| | 3.4 | 6 |
| 3 | 12.6 | 0 |
| | 14.2 | 1 |
| | 8.2 | 3 |
| | 8.2 | 6 |
| | | |
| 4 | 17.3 | 0 |
| | 8.9 | 1 |
| | 7.5 | 3 |
| | 7.0 | 6 |
| | | |

In next Table IX, there are depicted values of proteinuria evolution (g/24h) without patient 5.

| Proteinuria (g/24h) | Months |
|---|---|
| 2.84 | 1 |
| 2.06 | 2 |
| 1.56 | 3 |
| 1.98 | 4 |

**Table XI. Plasma uric acid as a whole excluding patient 5**

| Plasma Uric acid (mg/dl) | Months |
|---|---|
| 7.4 | 0 |
| 6.5 | 1 |
| 6.7 | 3 |
| 6.9 | 6 |

**Table XII. Restoration of glomerular filtration (hematuria)**

| Hematuria (red blood cells per high power field) | Months |
|---|---|
| 30 | 0 |
| - | 1 |
| 2 | 3 |
| 3 | 6 |

### VIII. Changes in Th1 cells during evolution

During the induction phase, a decrease in Th1 was observed. Interestingly, this effect was previously considered, because the normal Th1 activity when Th17 are supressed may be associated with a recrudescence of the colitis and the glomerulonephritis. This effect was observed in patients 1 and 4 (in patient 3, Th1 percentage was not quantifiable). (See Table XIII for data in patient 1)

The selective vitamin D receptor activation may play a role in the Th1 expression through the inhibition of the IL-6 and TGF-beta. After the decrease percentage of the Th1 during the induction phase a potent increase in the same Th1 percentage was observed. Because the month 0 (M0) and month 3 (M3) time points were associated with changes in proteinuria from 2.1 to 0.8g/24hours respectively (about 60% in proteinuria reduction) it could be concluded that this was not a escape phenomenon, but represented a change in the phenotype of these cells obtained after treating with the sequential therapy.

### IX. Changes in Treg cells during evolution

In tested patients no changes in the Treg percentage were observed, neither on induction phase nor in the maintenance one. This represents that the sequential treatment did not affect the Treg cells (See Table XIII, above, for data in patient 1). The changes in the percentage of Treg were considered not significant; however is clear that these cells did not decrease in our patient under sequential treatment. An important increase on Treg has been observed after vitamin D administration, so in our patients the evolution in this cells was a consequence of the addition of the secukinumab after the selective vitamin D receptor activation.

The changes of the Treg as a whole (see Table XIV below) indicates a weak benefit in this type of cells. The underline effect responsible of this effect may be related to the inhibition of the TGF-beta as a consequence of the selective vitamin D receptor activator. This induced condition is also interesting, due to the following reasons
1.- The no reduction of Treg in the patients are beneficial for the inhibition of the renal fibrosis, which is one of the most important histological benefit searched in proposed treatment schedule.
2.- The no reduction of Treg also may be reflecting that an increase in the Treg is no longer needed after the treatment with paricalcitol.

**Table XIV. Evolution of Treg cells as a whole during follow-up, excluding patient 5**

| Treg cells (% of CD44 positive cells) | Months |
|---|---|
| 4.7 | 0 |
| 5.3 | 1 |
| 4.8 | 3 |
| - | 6 |

### X. Changes in Th17 cells evolution

Changes in the Th17 cells could only be quantified in patient 1, the changes in patient 3 and 4 were unquantifiable. The progressive decrease in these cells are consistent with the previous report. The decrease in Th17 could be due to the proposed sequential therapy. It is well accepted that paricalcitol and secukinumab have the potential to decrease these cells (see data of patient 1 in Table XIII above).

### XI. Resetting balance Th17/Treg

The evolution of the Th17/Ttreg confirms the important benefit observed in patient 1. The inflammatory profile decreased in about 50% at month 3. It is important to remark that at the end of the induction phase, no important change was observed in this parameter, while it was important at month 3. This effect may be explained because the change in the expression in the Th17 cells, may have occurred as a consequence of conformational changes and not because functional changes (see data of patient 1 in Table XIII above).

### XII. Evolution of the thyroid hormonal axes

In patient 3, who also suffered from a primary autoimmune hypothyroidism, a surprising effect was observed in the Thyroid-stimulating hormone (TSH) and free thyroxine (T4) levels, where a normalization of a previously chronic bad-controlled thyroid function was achieved progressively. The increase in the free T4 levels clearly indicates that the hormonal release from the thyroid gland was improving progressively. This effect also implicates that even after a long thyroid damage the thyroid cells was capable of the restoring its functions. This fact was observed despite the time of follow-up, no changes in doses of replacement with levothyroxine was performed, and is also important that the patient increased his basal weight, condition that normally is associated with higher replacement T4 dosage needs.

Data are depicted in next tables XV and XVI.

**Table XV. Evolution of plasma TSH during follow-up in patient 3**

| Plasma TSH (uU/ml) | Months |
|---|---|
| 16.3 | 0 |
| 1.8 | 1 |
| 0.8 | 3 |
| 1.7 | 6 |

**Table XVI. Evolution of plasma free T4 during follow-up in patient 3**

| Plasma free T4 (ng/dl) | Months |
|---|---|
| 1.14 | 0 |
| 1.24 | 1 |
| 1.28 | 3 |
| 1.32 | 6 |

### Citation List

- Wang et al., "Rapid onset of inflammatory bowel disease after receiving secukinumab infusion" ACG case Rep J, 2018 5: p. e56.
- Satoh et al., "A case of refractory lupus nephritis complicated by psoriasis vulgaris that was controlled with secukinumab", Case Report Lupus (2018) 0, 1-5.
- Ochi et al., "A case of secondary IgA nephropathy accompanied by psoriasis treated with secukinumab", CEN Case Rep-2019 Apr 3. doi: 10.1007/s13730-019-00393-5.
- Wu et al., "The emerging role of pathogenesis of IgA nephropathy", Journal of Clinical Medicine-2018, vol. no. 7, 225.
- Hamour et al, Local IL-17 Production Exerts a Protective Role in Murine Experimental Glomerulonephritis, PlosOne 2015. 10(8): p.e0136238.
- Galvan et al., Paradoxical Role of IL-17 in Progression of Diabetic Nephropathy, J Am. Soc. Nephrol 2016. 27(3): p. 657-8.
- Rosendhal et al., "Adaptative immunity and IL-17A are not involved in the progression of chronic kidney disease after 5/5 nephrectomy in mice", Br J Pharmacol. 2018 Sep 30. doi: 10.1111/bph.14509.
- Turner et al., "The Th17 immune response in renal inflammation", Kidney International -2010, vol. no. 77, pp.: 1070-1075.

## Claims

1. A combination comprising:
a) an inhibitor of interleukin-17; and
b) a vitamin D receptor agonist.

2. The combination according to claim 1, wherein the inhibitor of IL-17 is an antibody or a fragment of said antibody, both of which specifically recognize one or more epitopes of a mammal IL-17 and being capable of blocking IL-17 activity.

3. The combination according to claim 2, wherein the inhibitor of IL-17 is secukinumab or a fragment thereof, said fragment specifically recognizing one or more epitopes of a mammal IL-17 and being capable of blocking IL-17 activity.

4. The combination according to any of claims 1-3, wherein the vitamin D receptor agonist is selected from the group consisting of cacitriol, paricalcitol, calcipotriene, tacalcitol, ercalcitriol any pharmaceutically acceptable salt or ester thereof, and combinations thereof.

5. The combination according to claim 4, wherein the vitamin D receptor agonist is the selective vitamin D receptor activator paricalcitol.

6. The combination according to any of claims 1-5, which comprises secukinumab and paricalcitol.

7. The combination according to any of claims 1-6 comprising:
a) an inhibitor of IL-17, and
b) a vitamin D receptor agonist, both as previously defined,
wherein the amount of a) and the amount of b) in combination are therapeutically effective.

8. A package or kit of parts comprising:
i) a first pharmaceutical or veterinary composition which comprises an amount of an inhibitor of IL-17 as defined in any of claims 1-7, together with one or more pharmaceutically or veterinary acceptable excipients or carriers; and
ii) a second pharmaceutical or veterinary composition which comprises an amount of vitamin D receptor agonist, together with one or more pharmaceutically or veterinary acceptable excipients or carriers;
wherein the first and second compositions are separate compositions, and wherein the amount of the compound of inhibitor of IL-17 of i) and the amount of vitamin D receptor agonist of ii) in combination are therapeutically effective.

9. A combination as defined in any of claims 1-7, or a package or kit of parts as defined in claim 8, for use in the treatment and/or prevention of an autoimmune and autoinflammatory concomitant disease or condition.

10. The combination as defined in any of claims 1-7, or a package or kit of parts as defined in claim 8, for use as defined in claim 9, wherein said autoimmune and autoinflammatory concomitant disease or condition is nephropathy IgA.

11. A combination as defined in any of claims 1-7, or a package or kit of parts as defined in claim 8, for use in the treatment and/or prevention of a cancer, said cancer optionally with metastatic behaviour, in which deregulation of immune system takes place.

12. A combination as defined in any of claims 1-7, or a package or kit of parts as defined in claim 8, for use in the treatment and/or prevention of proteinuria.

13. The combination as defined in any of claims 1-7, or a package or kit of parts as defined in claim 8, for use according to any of claims 9-12, wherein both the inhibitor of IL-17 and vitamin D receptor agonist are formulated for first administration of the vitamin D receptor agonist for a period of time comprised from 2 to 4 weeks before administration of the inhibitor of IL-17.

14. The combination as defined in any of claims 1-7, or a package or kit of parts as defined in claim 8, for use according to any of claims 9-13, wherein both inhibitor of IL-17 and vitamin D receptor agonist are formulated for sequentially administration according to the following schedule:
- a daily dose of vitamin D receptor agonist; and
- a weekly dose of inhibitor of IL-17.

15. The combination as defined in any of claims 1-7, or a package or kit of parts as defined in claim 8, for use according to claim 14, wherein the daily dose of vitamin D receptor agonist is comprised from 0.4 mg/day to 1 mg/day; and the weekly dose of inhibitor of IL-17 activity is comprised from 100 mg/week to 300 mg/week.
